(19) **Europäisches Patentamt**

**European Patent Office**

**Office européen des brevets**

(11) **EP 0 963 375 B1**

(12) **FASCICULE DE BREVET EUROPEEN**

(45) Date de publication et mention
de la délivrance du brevet:
**08.05.2002 Bulletin 2002/19**

(51) Int Cl.⁷: **C07F 7/22**, C08K 5/57

(21) Numéro de dépôt: **97952107.7**

(22) Date de dépôt: **18.12.1997**

(86) Numéro de dépôt international:
**PCT/FR97/02353**

(87) Numéro de publication internationale:
**WO 98/30568 (16.07.1998 Gazette 1998/28)**

(54) **MALEATES D'ORGANOETAIN POUR STABILISER DES POLYMERES THERMOPLASTIQUES**

ORGANOZINNMALEATE ZUR STABILISATION VON THERMOPLASTISCHEN POLYMEREN

ORGANOTIN MALEATES FOR STABILISING THERMOPLASTIC POLYMERS

(84) Etats contractants désignés:
**AT BE CH DE DK ES FI FR GB GR IE IT LI LU NL PT SE**

(30) Priorité: **09.01.1997 FR 9700146**

(43) Date de publication de la demande:
**15.12.1999 Bulletin 1999/50**

(73) Titulaires:
- **Atofina**
  **92800 Puteaux (FR)**
- **ATOFINA CHEMICALS, INC.**
  **Philadelphia PA 19102-3222 (US)**

(72) Inventeurs:
- **CUILLERET, Muriel**
  **F-69004 Lyon (FR)**

- **MOREL, Patrick**
  **F-69130 Ecully (FR)**
- **REILLY, James, L.**
  **Lansdale, PA 19446 (US)**
- **SCHIPPER, Peggy, S.**
  **Strafford, PA 19087 (US)**

(74) Mandataire: **Treuil, Claude et al**
**Atofina**
**Département Propriété Industrielle**
**4-8 cours Michelet**
**La Défense 10**
**92091 Paris La Défense Cedex (FR)**

(56) Documents cités:
FR-A- 2 032 739      US-A- 3 433 763
US-A- 4 231 949

**Description**

La présente invention a pour objet une composition constituée par des maléates d'organoétain de poids moléculaire élevé, ainsi que son procédé d'obtention.

L'invention concerne également les compositions de polymères thermoplastiques stabilisées thermiquement à l'aide de ladite composition à base de maléates d'organoétain de poids moléculaire élevé. Plus particulièrement, l'invention concerne des compositions de polymères de chlorure de vinyle, stabilisées, à l'aide de ladite composition à base de maléates d'organoétain de poids moléculaire élevé.

Un autre objet de l'invention concerne des compositions de polymères du chlorure de vinyle stabilisées par ladite composition de maléates d'organoétain de poids moléculaire élevé laquelle permet, en outre, de réduire les phénomènes de dépôts sur les équipements pouvant être observés lors de leur mise en oeuvre par extrusion. Le mot dépôt est plus communément connu par l'homme du métier sous le nom de plate-out.

Les maléates d'organoétain constituent une large famille, bien connue depuis plus de trente ans pour leur utilisation dans la stabilisation thermique des polymères halogénés tels que le polychlorure de vinyle (PVC) et le polychlorure de vinyle chloré (CPVC). De nombreuses références décrivent en particulier les alkyles maléates d'organoétain, représentés par la formule $(R^2)_2Sn(OOC-CH=CH-COOR^3)_2$ où $R^2$ représente un groupement méthyle, butyle, octyle ou lauryle et $R^3$ une chaîne hydrocarbonée contenant de 1 à 12 atomes de carbone et, de manière plus rare, de 13 à 22 atomes de carbone.

L'efficacité des maléates d'organoétain dans les compositions de PVC, notamment destinées à des articles à utilisation extérieure, est bien connue.

Ainsi, dans l'article intitulé "Worldwide Weathering of Polyvinyl Chloride", par Emery Szabo et Robert Lally ; Polymer and Engineering Science, avril 1975, Vol. 15, n° 4, on présente les résultats d'études d'exposition climatique à long terme. Les compositions stabilisantes utilisées étaient des compositions de savons de baryum/cadmium, des compositions de maléates d'organoétain et des compositions de mercaptoacétates d'organoétain. On note dans le résumé et les conclusions que le DBTM (maléate de dibutylétain) donne les meilleurs résultats en tant que stabilisant UV et que les mercaptoacétates d'organoétain donnent les résultats les plus médiocres.

Bien que la supériorité des compositions de maléates d'organoétain soit depuis longtemps reconnue pour la stabilisation de formulations de PVC soumises au vieillissement climatique, ces compositions n'ont pas réussi à satisfaire pleinement les besoins du marché en raison de nombreux inconvénients.

Ainsi, le brevet britannique 787 930 explique de la page 1 ligne 65 à la page 2 ligne 3, que le maléate de dibutylétain est difficile à disperser, et génère en outre lors de la transformation du PVC une fraction volatile d'anhydride maléique provoquant des effets lacrymogènes et irritants sur les personnes le manipulant. Afin de surmonter ces défauts, il est proposé dans le GB 787 930 des compositions d'hémiesters maléates d'organoétain liquides, telles que le bis(monobutylmaléate) de dibutylétain. La présence du groupement alcool de l'ester a pour effet de diminuer la teneur en étain, entraînant une stabilisation thermique moins importante, mais n'élimine pas la fraction volatile générée lors du mécanisme de stabilisation, c'est-à-dire la formation de l'hémiester d'acide maléique par la réaction du bis (monoalkylmaléate) d'organoétain avec l'HCl gazeux généré pendant la mise en oeuvre du PVC.

Un autre problème qui se pose par l'utilisation des maléates d'organoétain comme stabilisant thermique des polymères de chlorure de vinyle est la difficulté de mise en oeuvre de ces formulations. Il est en effet bien connu, notamment lors de l'extrusion de PVC rigide, que le polymère fondu contenant les dits stabilisants présente une forte tendance au collage sur les équipements de transformation.

Pour remédier à ce problème, on utilise des combinaisons parfois complexes de lubrifiants, et on essaie plus rarement de modifier la structure du stabilisant lui-même.

Ainsi, le brevet GB 1378851 propose l'addition d'un mélange de cire de paraffine et d'un polymère acrylique lubrifiant à une formulation PVC contenant du dibutyl diméthylmaléate d'étain, de manière à réduire le collage sur les équipements. L'amélioration ainsi obtenue est caractérisée par une plus grande facilité à décoller un film transformé sur un malaxeur à cylindre, mais reste toutefois limitée.

La tendance prononcée au collage des maléates d'organoétain, classiquement utilisés par les transformateurs de PVC, peut aussi se traduire par l'apparition de dépôts important dans les équipements de transformation et particulièrement à l'entrée des filières d'extrusion. De nombreuses publications et brevets proposent d'y remédier en associant aux maléates d'organoétain différents types de molécules organiques.

Ainsi, la demande de brevet japonais 61296048 démontre que l'association d'un maléate de dibutylétain avec un savon métallique de chaîne hydrocarbonée pouvant aller jusqu'à 29 atomes de carbone, le métal étant choisi parmi le magnésium, le calcium, le strontium, le baryum ou le zinc, permet d'obtenir des formulations PVC présentant un plate-out réduit.

Dans la demande de brevet japonais 61209248, il est clairement mis en évidence lors de l'extrusion de plaques, qu'un acide mercaptopropionique substitué additionné à une formulation stabilisée par le di(éthylhexylmaléate) de dibutylétain permet de supprimer totalement le plate-out.

**EP 0 963 375 B1**

On peut encore voir dans la demande de brevet japonnais 04285651 qu'une combinaison d'un hémiester de l'acide orthophtalique comprenant de 1 à 12 atomes de carbone et de butylstéarate permet de réduire le plate-out d'une formulation PVC stabilisée avec le maléate de dibutylétain.

Cependant, l'addition importante voire excessive de lubrifiants aux formulations PVC, comme il est le plus souvent proposé, peut présenter des inconvénients. Ainsi, une teneur trop importante en lubrifiants internes aura entre autres conséquences, d'augmenter la plastification du PVC et donc de diminuer son point Vicat. Dans le cas de formulations rigides, ceci peut constituer un élément réhibitoire à l'utilisation des maléates d'organoétain.

De la même manière, un excès de lubrifiants externes destinés à limiter les problèmes de collage lors de l'extrusion, aura tendance à favoriser les phénomènes d'exsudation et donc de dépôts sur les équipements.

FR 2032739 décrit des maléates d'organoétain utilisés comme stabilisants thermiques et possédant de propriétés lubrifiantes.

Ainsi, malgré plus de trente années de recherches, les stabilisants thermiques du PVC à base de maléates d'organoétain continuent d'avoir une utilisation limitée bien qu'ils présentent d'excellentes propriétés stabilisantes, et une très bonne résistance au vieillissement climatique. Leurs propriétés ne peuvent être considérées comme entièrement satisfaisantes :

- soit parce que lors de la mise en oeuvre, ils se décomposent en produits volatiles irritants et lacrymogènes,
- soit parce qu'ils sont difficiles à mettre en oeuvre, donnant en particulier des formulations ayant une forte tendance au collage et pouvant entraîner des problèmes de dépôts sur les équipements de transformation.

On a maintenant trouvé une composition de maléates d'organoétain de poids moléculaire élevé qui peut être obtenue en faisant réagir, éventuellement en milieu solvant et/ou en présence d'eau, un composant RA avec l'anhydride maléique ou l'acide maléique, puis en mettant en contact le milieu réactionnel ainsi obtenu avec au moins un oxyde de dialkyl étain $(R^1)_2$ Sn = O ou avec au moins un chlorure d'alkylétain $(R^1)_x SnCl_{4-x}$, étant donné que :

RA représente

- soit un alcool ROH dans lequel R représente un radical hydrocarboné aliphatique, linéaire ou ramifié, ayant un nombre d'atomes et de carbone allant de 23 à 50, ou un mélange d'alcools primaires saturés de masse moléculaire moyenne en poids $\overline{Mw}$ allant de 340 à 718, et une polydispersité $\overline{Mw}/\overline{Mn}$ voisine de 1 ($\overline{Mn}$ représentant la masse moléculaire moyenne en nombre),
- soit un époxyalcane $C_nH_{2n}O$ dans lequel n va de 23 à 50 ou un mélange d'époxyalcanes de masse moléculaire moyenne en poids $\overline{Mw}$ allant de 338 à 716 ;

$R^1$ représente un radical hydrocarboné aliphatique, linéaire ou ramifié, ayant un nombre d'atomes de carbone allant de 1 à 12, et de préférence égal à 1,4 ou 8,
x est un nombre entier égal à 1 ou 2.

Le polymère thermoplastique dans lequel on peut incorporer la composition à base de maléates d'organoétain de poids moléculaire élevé en vue d'en améliorer notamment la stabilité thermique et la stabilité à la lumière peut notamment consister en un ou plusieurs polymères d'addition choisis dans le groupe formé par les homopolymères du chlorure de vinyle qui peuvent être éventuellement surchlorés, et les copolymères, éventuellement greffés, qui résultent de la copolymérisation du chlorure de vinyle avec un ou plusieurs comonomères éthyléniquement insaturés. A titre de comonomères pour la préparation de tels copolymères conviennent notamment les halogénures de vinylidène comme le chlorure ou le fluorure de vinylidène, les carboxylates de vinyle comme l'acétate de vinyle, le propionate de vinyle, le butyrate de vinyle, les acides acrylique et méthacrylique ainsi que les nitriles, amides et esters d'alkyle qui en dérivent, notamment acrylonitrile, acrylamide, méthacrylamide, méthacrylate de méthyle, acrylate de méthyle, acrylate de butyle, acrylate d'éthyle, acrylate d'éthyl-2 hexyle, les dérivés vinylaromatiques tels que styrène, vinylnaphtalène, les oléfines telles que bicyclo(2.2.1)hept-2-ène, bicyclo(2.2.1)hepta-2,5-diène, éthylène, propène, butène-1.

Parmi ces polymères, l'invention concerne tout particulièrement les homo- et copolymères du chlorure de vinyle, éventuellement surchlorés.

La composition à base de maléates d'organoétain de poids moléculaire élevé selon la présente invention peut être préparée selon le mode préféré ci-après qui consiste à fondre, en milieu solvant et en chauffant, le composant RA. Ensuite, au milieu ainsi obtenu, porté à une température au moins égale à 80°C et, de préférence, comprise entre 90°C et 100°C, on ajoute d'abord l'anhydride maléique, en continu ou par portion, en une durée qui peut aller de 15 minutes à environ 1 heure, puis ensuite un oxyde de dialkylétain $(R^1)_2Sn=O$ en continu ou par portion, en une durée qui est au moins égale à 15 minutes, et, de préférence, allant de 30 minutes à 90 minutes.

Le milieu réactionnel est ensuite maintenu à une température allant de 80°C à 120°C et, de préférence,

comprise entre 90°C et 100°C, pendant une durée qui est au moins égale à 15 minutes et, de préférence, allant de 30 minutes à 90 minutes.

L'eau formée au cours de la réaction ainsi que le solvant peuvent être éliminés par distillation sous pression réduite à une température allant de 80°C à 120°C et, de préférence comprise entre 90°C et 100°C.

Le solvant utilisé doit être inerte vis-à-vis des réactifs et des produits formés.

A titre d'illustration de solvants utilisables selon la présente invention on citera, le toluène, les xylènes, l'heptane, le THF.

Selon la présente invention, on opère sous agitation et, de préférence sous bullage de gaz inerte tel que l'azote.

On opère généralement à pression atmosphérique ($10^5$Pa), mais on ne sortirait pas du cadre de l'invention si on opérait à une pression différente.

Dans l'éventualité où l'on utilise des époxyalcanes, on opérera en présence d'eau. Celle-ci peut être avantageusement introduite avant l'addition de l'anhydride maléique.

Selon la présente invention, on utilisera le composant RA ainsi que l'anhydride maléique dans un rapport molaire anhydride maléique/RA au moins égal à 2 et, de préférence compris entre 2,5 et 4.

A titre d'illustration de composants RA utilisables selon la présente invention, on citera les alcools ROH tels que le 1-nonacosanol (alcool montanylique), le hentriacontanol, le n-triacontanol (alcool myricylique), les mélanges d'alcools primaires saturés ayant une masse moléculaire moyenne en poids $\overline{Mw}$ allant de 375 à 700, une polydispersité $\overline{Mw}/\overline{Mn}$ voisine de 1 et une distribution régulière des radicaux hydrocarbonés aliphatiques linéaires en fonction de la longueur des chaînes hydrocarbonées, les mélanges de 1,2-époxyalcanes ayant une masse moléculaire moyenne en poids $\overline{Mw}$ allant de 338 à 632.

On utilisera de préférence un mélange d'alcools primaires saturés ayant une masse $\overline{Mw}$ égale à 460 ou 550, ou un mélange d'époxyalcanes ayant une masse $\overline{Mw}$ égale à 632.

Selon la présente invention, on utilisera, pour 1 mole d'anhydride maléique au moins 0,50 mole d'oxyde de dialkylétain $(R^1)_2Sn=O$ et, de préférence, une quantité allant de 0,75 mole à 0,85 mole.

A titre d'illustration d'oxydes de dialkylétain utilisables selon la présente invention, on citera l'oxyde de diméthylétain, l'oxyde de dibutylétain, l'oxyde de dioctylétain.

Parmi ces composés, on préfère utiliser tout particulièrement l'oxyde de dibutylétain.

On peut également utiliser lors de la préparation de la composition à base de maléates d'organoétain de poids moléculaire élevé au moins un chlorure d'alkylétain $(R^1)_xSnCl_{4-x}$ à la place d'un oxyde de dialkylétain $(R^1)_2Sn=O$.

De préférence, on utilisera un mélange de trichlorure de monoalkylétain $R^1SnCl_3$ et de dichlorure de dialkylétain $(R^1)_2SnCl_2$ ; les radicaux $R^1$ du trichlorure et du dichlorure pouvant être identiques ou différents.

Dans ce cas, les chorures formés lors de la préparation de la composition peuvent être neutralisés par un hydroxyde alcalin.

La composition à base de maléates d'organoétain de poids moléculaire élevé selon la présente invention possède une teneur pondérale en étain au moins égale à 10 % et, de préférence, comprise entre 15 % et 25 %.

Dans le cas général où les compositions obtenues sont solides à température ambiante, celles-ci peuvent être isolées par les moyens connus de l'homme du métier tels que notamment par coulage du milieu réactionnel chaud et liquide sur une surface refroidie puis écaillage du produit solidifié.

Sur la composition obtenue, on peut déterminer la teneur en étain par analyse élémentaire. Le spectre infrarouge de la composition selon l'invention se caractérise par :

- une bande d'absorption aux environs de 1725 cm$^{-1}$, caractéristique des fonctions esters,
- une bande d'absorption aux environs de 1585 cm$^{-1}$, caractéristique des carboxylates d'étain,
- une bande d'absorption aux environs de 680 cm$^{-1}$, caractéristique des liaisons $\equiv$ Sn — O — Sn $\equiv$.

La composition à base de composés organiques de l'étain de poids moléculaire élevé peut être obtenue selon une variante qui consiste à introduire après l'élimination de l'eau formée, et éventuellement du solvant, une quantité de costabilisant au moins égale à 10 % en poids et, de préférence, comprise entre 15 % et 30 %, par rapport aux réactifs mis en oeuvre (hors eau).

Cette additon effectuée, on maintient le milieu réactionnel sous agitation et chauffage jusqu'à obtention d'un mélange homogène.

A titre d'illustration de costabilisants utilisables selon la présente invention, on citera les zéolithes, les hydrotalcites, les sels d'acides gras de calcium et de zinc.

L'invention a également pour objet un procédé d'obtention de maléates d'organoétain de poids moléculaire élevé caractérisé en ce que l'on fait réagir un composant RA avec l'anhydride maléique, puis que l'on met en contact le milieu réactionnel ainsi obtenu avec au moins un oxyde de dialkylétain $(R^1)_2Sn = O$ ou avec au moins un chlorure d'alkylétain $(R^1)_xSnCl_{4-x}$. RA, $(R^1)_2Sn = O$ et $(R^1)_xSnCl_{4-x}$ ont la même signification que précédemment donnée. Les conditions et paramètres opératoires, les réactifs utilisés et leurs proportions sont tels que définis ci-avant.

L'invention concerne également les compositions comprenant le polymère thermoplastique et la composition à base de maléates d'organoétain de poids moléculaire élevé tels que définis ci-avant.

La composition à base de maléates d'organoétain de poids moléculaire élevé peut être utilisée en des quantités allant de 0,5 et 5 parties en poids, de préférence de 1 à 4 parties, pour 100 parties en poids de polymère thermoplastique.

De telles compositions peuvent renfermer en outre, et en fonction notamment des conditions de mise en oeuvre ou de transformation et/ou des applications auxquelles elles sont destinées, les additifs usuels tels que pigments, charges, lubrifiants, agents de mise en oeuvre, modifiants choc, antioxydants, plastifiants et agents gonflants.

La composition à base d'étain de poids moléculaire élevé telle qu'obtenue selon la présente invention peut être incorporée, en même temps, ou avant les additifs mentionnés précédemment lorsqu'il en est fait usage.

Selon une modalité particulièrement recommandée, on effectue cette opération dans un mélangeur rapide et on introduit successivement le polymère thermoplastique, la composition à base de maléates d'étain de poids moléculaire élevé, puis les additifs et les charges.

En règle générale, cette opération peut s'effectuer à température ambiante, l'opération elle-même pouvant provoquer une élévation de température par exemple jusqu'à 70°C, voire davantage.

L'action stabilisante de la composition à base de maléates d'organoétain de poids moléculaire élevé, telle qu'obtenue selon la présente invention, peut être mise en évidence en effectuant différents tests évaluant la stabilité thermique dynamique et statique des compositions de polymère thermoplastique la contenant.

Les compositions de polymères thermoplastiques, notamment de PVC, comprenant la composition à base de maléates d'organoétain de poids moléculaire élevé, telle qu'obtenue selon la présente invention peuvent être moulées par injection, calandrées puis thermoformées, extrudées, coextrudées en articles rigides tels que revêtement intérieurs pour le bâtiment, huisseries, profilés de fenêtre, feuilles et tubes. Les articles transformés peuvent être compacts ou expansés.

L'action stabilisante à la chaleur et la lumière de la composition à base d'organoétain de poids moléculaire élevé, peut être mise en évidence pour les articles transformés par une mesure des coordonnées trichromatiques L*, a*, b*, le temps de stabilité résiduelle par un test de Rouge Congo et en soumettant ces articles aux rayonnements UV.

La composition de maléates d'organoétain de poids moléculaire élevé telle qu'obtenue selon la présente invention présente les avantages, outre de stabiliser thermiquement les compositions de polymères thermoplastiques la contenant, de leur apporter un effet lubrifiant particulièrement significatif. Cette caractéristique peut être mise en évidence en étudiant la rhéologie à l'aide d'un rhéomètre à couple de torsion, ou par la lecture des paramètres de transformation lors de la mise en oeuvre. On a ainsi constaté que l'effet lubrifiant apporté par la composition de maléates d'organoétain de poids moléculaire élevé de la présente invention permet de réduire de manière significative l'addition de lubrifiants dans les compositions de PVC.

La composition de maléates d'organoétain de poids moléculaire élevé telle qu'obtenue selon la présente invention présente également l'avantage de ne pas altérer le point VICAT des compositions de polymères thermoplastiques transformées.

De plus, lors de la transformation des compositions de polymères thermoplastiques contenant ladite composition de maléates d'organoétain de poids moléculaire élevé , et plus particulièrement en extrusion, on n'observe pas de dégagement de produits irritants.

On a constaté que l'utilisation des composants RA ayant un nombre d'atomes de carbone supérieur à 22, et plus particulièrement supérieur à 30 permettait de limiter, voire d'éviter les problèmes de plate-out observés avec des composants RA ayant un nombre d'atomes de carbone inférieur. En effet, on a observé que l'utilisation, dans des compositions de maléates d'organoétain de structure comparable à celle décrite dans l'invention, de composants RA, et plus précisément d'alcools, ayant un nombre d'atomes de carbone compris entre 16 et 22 conduisait, lors de l'extrusion de formulations PVC, à des dépôts de l'alcool gras correspondant sur les conformateurs des extrudeuses. Ceci a pu être mis en évidence en observant puis en analysant les dépôts sur la surface des conformateurs après une durée d'extrusion donnée. Dans les mêmes conditions opératoires, on a observé que les compositions de PVC contenant les compositions de maléates d'organoétain de poids moléculaire élevé décrites dans la présente invention ne conduisent à aucun dépôt sur les calibrateurs.

Les exemples qui suivent illustrent l'invention.

**I Préparation des compositions de maléates d'organoétain de poids moléculaire élevé conformes à l'invention**

Les compositions de maléates d'organoétain ont été préparées en utilisant les réactifs suivants :

- UNILIN®425 (commercialisé par la Société PETROLITE) : mélange d'alcools primaires saturés ayant une masse moléculaire moyenne en poids $\overline{Mw}$ égale à 460, une polydispersité $\overline{Mw}/\overline{Mn}$ égale à 1, présentant un point de fusion de 91°C, un nombre d'hydroxyle égal à 105 mg KOH/g d'échantillon déterminé selon la norme ASTM D 222 et un nombre moyen d'atomes de carbone moyen égal à 30.

- UNILIN®550 (commercialisé par la Société PETROLITE) :

   mélange d'alcools primaires saturés ayant une masse moléculaire moyenne en poids $\overline{Mw}$ égale à 550, une polydispersité $\overline{Mw}/\overline{Mn}$ égale à 1, présentant un point de fusion de 99°C, un nombre d'hydroxyle égal à 83 mg KOH/g d'échantillon déterminé selon la norme ATSM D 222 et un nombre moyen d'atomes de carbone moyen égal à 40.

- VIKOLOX®C30+ (commercialisé par la société ELF ATOCHEM NORTH AMERICA Inc.) : mélange de 1,2-époxyalcanes ayant une masse moléculaire en poids $\overline{Mw}$ égale à 632 et présentant un point de fusion de 72,7°C et un nombre moyen d'atomes de carbone moyen égal à 45.
- VALFOR®100 (commercialisé par la société The PQ Corporation) : Zeolithe A de type aluminosilicate de sodium hydraté ;
- anhydride maléique,
- oxyde de dibutylétain

**EXEMPLE 1 :**

**PREPARATION D'UNE COMPOSITION DE MALEATES D'ORGANOETAIN DE POIDS MOLECULAIRE ELEVE SELON L'INVENTION, EN MILIEU SOLVANT.**

Dans un réacteur double enveloppe de 500 ml, muni d'une agitation, d'une gaine thermométrique, d'un condenseur équipé d'un Dean-Stark et d'une arrivée de gaz inerte, on introduit :

- 100 ml d'heptane (solvant),
- 91,6 g (0,199 mole) d'Unilin® 425,

Le mélange est chauffé vers 90°C et agité de manière à obtenir un milieu réactionnel liquide et homogène. Sous courant d'azote, on introduit 53,6 g (0,546 mole) d'anhydride maléique, en quatre portions sur 30 minutes, provoquant un léger exotherme à 100°C. Lorsque la température se stabilise à nouveau vers 90°C, on ajoute 109,5 g (0,440 mole) d'oxyde de dibutylétain en cinq portions sur 1 heure. Le milieu réactionnel est maintenu sous agitation et sous courant d'azote pendant 1 heure à 90°C. On augmente la température de 5°C à 10°C avant d'appliquer une pression de $6,66.10^3$ Pa. afin de distiller l'heptane et l'eau de réaction (environ 1,8 g d'eau). Après 1 heure de distillation, le réacteur est remis sous pression atmosphérique. On stoppe le chauffage et l'agitation, puis on coule le milieu réactionnel sur une plaque métallique refroidie de façon à le solidifier.

On obtient environ 251 g de la composition soit un rendement pondéral voisin de 99 %. Le pourcentage pondéral d'étain de la composition ainsi obtenue est de 20,7 %. Le spectre infra-rouge présente une bande d'absorption à 1726 cm$^{-1}$ correspondant aux fonctions esters, des bandes d'absorption à 1589 cm$^{-1}$ correspondant aux fonctions carboxylate d'étain -C(O)-O-Sn≡ et une bande d'absorption à 677 cm$^{-1}$ caractéristique des liaisons ≡Sn-O-Sn≡.

**EXEMPLE 2 :**

**PREPARATION D'UNE COMPOSITION DE MALEATES D'ORGANOETAIN DE POIDS MOLECULAIRE ELEVE SELON L'INVENTION, UTILISANT UNE ZEOLITHE COMME COSTABILISANT.**

On opère comme dans l'exemple 1 en utilisant les mêmes réactifs selon des quantités identiques. Ainsi on utilise :

- 100 ml d'heptane (solvant),
- 91,6 g (0,199 mole) d'Unilin® 425,
- 53,6 g (0,546 mole) d'anhydride maléique,
- 109,5 g (0,440 mole) d'oxyde de dibutylétain.

Après l'élimination de l'heptane et de l'eau de réaction par distillation sous pression réduite, on remet le réacteur sous pression atmosphérique et on introduit 64 g de Valfor®100 en quatre portions sur 30 minutes, sous agitation et en maintenant la température à 90°C. Après homogénéisation du milieu réactionnel le produit est isolé comme dans l'exemple 1.

On obtient environ 316 g de la composition soit un rendement pondéral voisin de 100 %. Le pourcentage pondéral d'étain de la composition ainsi obtenue est de 16,5 %. Le spectre infra-rouge présente des bandes similaires à celles de la composition de l'exemple 1.

**EXEMPLE 3**

**PREPARATION D'UNE COMPOSITION SELON L'INVENTION, UTILISANT L'UNILIN ®550 EN REMPLACEMENT DE L'UNILIN®425.**

On opère comme dans l'exemple 2 en augmentant de 5°C les consignes de température et en utilisant les réactifs selon les quantités suivantes :

- 100 ml d'heptane (solvant),
- 91,6 g (0,167 mole) d'UNILIN®550,
- 53,6 g (0,546 mole) d'anhydride maléique,
- 109,5 g (0,440 mole) d'oxyde de dibutylétain.

Après l'élimination de l'heptane et de l'eau de réaction par distillation sous pression réduite, on remet le réacteur sous pression atmosphérique et on introduit 64 g de Valfor®100 en quatre portions sur 30 minutes, sous agitation et en maintenant la température à 95°C. Après homogénéisation de milieu réactionnel, le produit est isolé comme dans l'exemple 1.

On obtient environ 316 g de la composition soit un rendement pondéral voisin de 100 %. Le pourcentage pondéral d'étain de la composition ainsi obtenue est de 16,5 %. Le spectre infra-rouge présente des bandes d'absorption similaires à celles de la composition de l'exemple 2.

**EXEMPLE 4**

**PREPARATION D'UNE COMPOSITION SELON L'INVENTION, UTILISANT UN MELANGE DE 1,2-EPOXYALCANES, EN PRESENCE D'EAU ET EN ABSENCE DE SOLVANT.**

On opère selon les conditions opératoires de l'exemple 1, dans un réacteur identique, avec les quantités de réactifs suivantes :

- 25 g d'eau,
- 91,6 g (0,145 mole) de Vikolox®C30+,
- 53,6 g (0,546 mole) d'anhydride maléique,
- 109,5 g (0,440 mole) d'oxyde de dibutylétain.

La distillation sous pression réduite s'effectue de façon similaire à l'exemple 1 et on recueille 21 g d'eau après 1 heure. On introduit alors 64 g de Valfor®100 dans le milieu réactionnel comme décrit dans l'exemple 2.

Le produit est recueilli comme dans les exemples 1 et 2. On obtient envrion 322 g de la composition soit un rendement pondéral voisin de 100 %. Le pourcentage pondéral d'étain de la composition ainsi obtenue est de 16,2 %.

Le spectre infra-rouge présente une bande d'absorption à 1726 cm$^{-1}$ correspondant aux fonctions esters, une bande d'absoption à 1581 cm$^{-1}$ correspondant aux fonctions carboxylates d'étain -C(O)-O-Sn≡ et une bande d'absorption à 677 cm$^{-1}$ caractéristique des liaisons ≡Sn-O-Sn≡.

**Il Préparation des compositions de PVC, ci-après désignées par formulations PVC, contenant les compositions de maléates d'organoétain de poids moléculaire élevé précédemment préparées et évalutation des propriétés stabilisantes thermiques desdites compositions de maléates d'organoétain.**

Ci-après, on donne les noms commerciaux, les natures, les fournisseurs et les fonctions des différentes matières utilisées pour la préparation des formulations de PVC.

- LACOVYL S11OP, résine PVC de K Wert égal à 67, ELF ATOCHEM S.A.,
- DURASTRENGTH 300, polymère acrylique, CECA S.A., modifiant choc,
- STAVINOR CAPSE, stéarate de calcium, CECA S.A., lubrifiant,
- METABLEN P551, polymère acrylique, METABLEN COMPANY B.V., agent de mise en oeuvre,
- KRONOS S2220, oxyde de titane, KRONOS, pigment,

- HYDROCARB 95T, carbonate de calcium, OMYA S.A., charge
- AC 316, cire de polyéthylène oxydée, ALLIED SIGNAL, lubrifiant.

ON PREPARE LES FORMULATIONS PVC SELON LES CONDITIONS OPERATOIRES SUIVANTES (LES QUANTITES DES MATIERES UTILISEES SONT EXPRIMEES EN POIDS) :

On introduit dans un mélangeur rapide Henschel muni d'une double enveloppe, avec une vitesse d'agitation de 3 800 tours/minute :

- 100 parties de résine PVC (Lacovyl S 110 P).

On observe une élévation de température.

A 60°C, on introduit 3,5 parties d'une composition de maléates d'organoétain de poids moléculaire élevé, telle qu'obtenue selon les exemples 1 à 4, puis à 65°C, on introduit 0,2 partie de cire de polyéthylène oxydée (AC 316).

A 80°C, on ajoute :

- 0,6 partie de stéarate de calcium (Stavinor CA PSE),
- 7,5 parties de modifiant choc (Durastrength 300) et,
- 1,5 parties d'agent de mise en oeuvre (Metablen P 551).

Puis à 85°C, on ajoute :

- 4 parties d'oxyde de titane (Kronos S2220),
- 5 parties de carbonate de calcium (Hydrocarb 95 T).

La formulation PVC est soumise à une agitation de 3 800 tours/minute jusqu'à atteindre une température de 110°C, puis refroidie en réduisant l'agitation à 1500 tours/minute et en faisant circuler un courant d'eau froide dans la double-enveloppe du mélangeur.

La formulation obtenue est recueillie lorsque la température atteint environ 45°C.

On réalise un test de stabilité thermique dynamique sur trois formulations PVC.

On désigne ci-après par :

- Formulation PVC 1, une formulation contenant la composition de maléates d'organoétain de poids moléculaire élevé de l'exemple 2 ;
- Formulation PVC 2, une formulation contenant la composition de maléates d'organoétain de poids moléculaire élevé de l'exemple 3 ;
- Formulation PVC 3,une formulation contenant la composition de maléates d'organoétain de poids moléculaire élevé de l'exemple 4.

150 g des formulations PVC 1, 2 et 3 sont évaluées à l'aide d'un malaxeur à cylindre COLLIN, dont les rouleaux sont portés à 200°C. Les vitesses de rotation des deux cylindres sont respectivement réglées à 20tr/min et 24 tr/min de manière à gélifier puis travailler la matière entre les cylindres en apportant un travail mécanique de friction. L'écartement entre les cylindres est réglé à 0,7 mm.

Des échantillons sont prélevés sur les cylindres à intervalle de temps régulier en notant leur coloration jusqu'à dégradation totale.

Les indices de jaune (YI, norme ASTM E313) mesurés sur chaque échantillon prélevé, ainsi que le temps de dégradation correspondant au noircissement total sont présentés dans le tableau 1.

|  | Formulation PVC 1 | Formulation PVC 2 | Formulation PVC 3 |
|---|---|---|---|
| YI (2 min) | 2,4 | 2,9 | 3,4 |
| YI (4 min) | 4,0 | 3,7 | 3,4 |
| YI (6 min) | 5,4 | 5,8 | 5,5 |
| YI (8 min) | 7,3 | 7,1 | 6,6 |
| YI (10 min) | 8,4 | 8,2 | 8,5 |
| YI (12 min) | 9,0 | 8,9 | 9,2 |
| YI (15 min) | 9,8 | 10,5 | 11,0 |
| YI (20 min) | 12,3 | 12,4 | 14,0 |
| YI (25 min) | 14,9 | 16,2 | 17,2 |
| YI (30 min) | 17,9 | 18,7 | 21,0 |
| YI (40 min) | 26,6 | 27,2 | 29,0 |
| YI (50 min) | 31,4 | 31,6 | 33,0 |
| Dégradation totale (min) | > 60 | > 60 | > 60 |

**TABLEAU 1**

Les résultats présentés mettent en évidence l'action remarquable des compositions de maléates d'organoétain de poids moléculaire élevé décrites dans l'invention comme stabilisant thermique du PVC.

**III EVALUATION DES PROPRIETES FINALES DE PROFILES RIGIDES EN PVC FORMES A PARTIR DE FORMULATIONS PVC CONTENANT LES COMPOSITIONS DE MALEATES D'ORGANOETAIN DE POIDS MOLECULAIRE ELEVE ET MISE EN EVIDENCE DE LA (REDUCTION) SUPPRESSION DU PLATE-OUT APPORTE PAR LESDITES COMPOSITIONS.**

Afin de mettre en évidence les propriétés finales des profilés rigides stabilisés thermiquement par les compositions de maléates d'organoétain de poids moléculaire élevé décrites dans l'invention ainsi que l'apport desdites compositions à la (réduction) suppression du plate-out, on a extrudé, parallèlement aux formulations PVC stabilisées par les compositions de maléates d'organoétain de poids moléculaires élevés de la présente invention des compositions de maléates d'organoétain obtenues avec des alcools primaires linéaires ayant un nombre d'atomes de carbone égal ou inférieur à 22.

On désigne par :

- Formulation PVC 4, une formulation contenant une composition de maléates d'organoétain obtenue selon les conditions opératoires de l'exemple 2 et avec les mêmes quantités pondérales de réactifs excepté que l'on remplace l'UNILIN®425 par l'alcool stéarique(1-octadécanol).
- Formulation PVC 5, une formulation contenant une composition de maléates d'organoétain obtenue selon les conditions opératoires de l'exemple 2 et avec les mêmes quantités pondérales de réactifs excepté que l'on remplace l'UNILIN®425 par l'alcool béhénique(1-docésanol).

Ces deux formulations PVC 4 et 5 sont préparées selon le protocole utilisé pour préparer les formulations PVC 1, 2 et 3 (mêmes conditions opératoires, mêmes quantités pondérales des matières utilisées).

Les cinq formulations PVC sont extrudées sur une extrudeuse KMDL25 équipée d'une filière plate 60 x 1 mm. A la sortie de la filière, le matériau est calibré dans un conformateur maintenu à une température de 15°C grâce à une circulation d'eau froide, et équipé d'une pompe à vide afin d'assurer la mise en forme finale du profilé ainsi obtenu.

Pour chacune des formulations PVC 1 à 5, on effectue une extrusion durant une heure. Les paramètres de transformation sont détaillés dans le tableau 2.

| | Formulation PVC 1 | Formulation PVC 2 | Formulation PVC 3 | Formulation PVC 4 (comparatif) | Formulation PVC 5 (comparatif) |
|---|---|---|---|---|---|
| Profilé de température (°C) | 140 - 190 - 190 - 200 | | | | |
| Température masse (°C) | 191 | 190 | 191 | 191 | 192 |
| Vitesse moteur (tr/min) | 16 | 15 | 15 | 16 | 16 |
| Couple (%) | 32 | 30 | 26/27 | 45 | 41 |
| Pression tête (bar) | 175 | 160 | 146 | 200 | 195 |
| Débit (kg/h) | 4,8 | 4,9 | 4,9 | 4,1 | 4,2 |

**TABLEAU 2**

Après chaque extrusion, le calibrateur est ouvert et sa surface est observée.

Aucun dépôt n'est observé pour les formulations PVC 1 à 3 contenant les compositions de maléates d'organoétain de poids moléculaire élevé de la présente invention.

A l'inverse, les formulations PVC 4 et 5 donnent lieu à l'apparition d'un dépôt blanc gras. On observe très clairement que le dépôt entraîné par l'extrusion de la formulation PVC 4, contenant le stabilisant utilisant l'alcool stéarique, est plus important en masse que le dépôt entraîné par l'extrusion de la formulation PVC 5, contenant le stabilisant utilisant l'alcool béhénique.

L'analyse des dépôts a été effectuée par analyse infra-rouge ; elle met en évidence que ces dépôts sont constitués de manière exclusive par l'alcool utilisé lors de la préparation de la composition de maléates d'organoétain, à savoir :

- alcool stéarique dans le cas de la formulation PVC 4,
- alcool béhénique dans le cas de la formulation PVC 5.

La photographie 1 illustre les phénomènes de dépôt observés dans le calibrateur après une heure d'extrusion avec la formulation PVC 4.

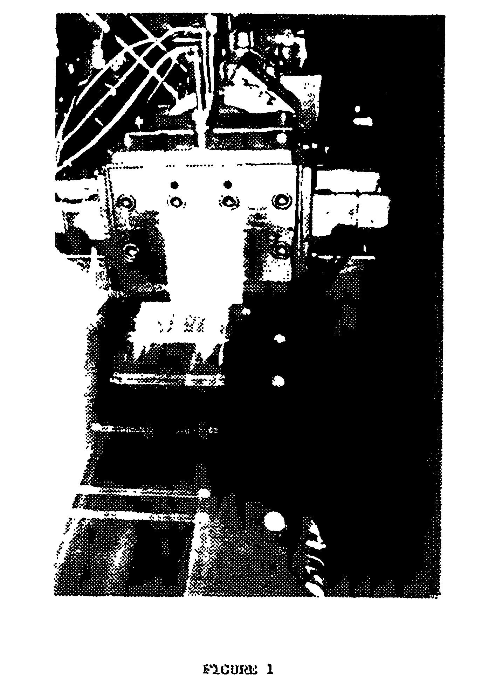

**Revendications**

1. Composition de maléates d'organoétain de poids moléculaire élevé telle qu'obtenue par la réaction d'un composant RA avec l'anhydride maléique, puis mise en contact du milieu réactionnel ainsi obtenu avec au moins un oxyde de dialkyl étain $(R^1)_2$ Sn = O ou avec au moins un chlorure d'alkylétain $(R^1)_x SnCl_{4-x}$, étant donné que :

RA représente

- soit un alcool ROH dans lequel R représente un radical hydrocarboné aliphatique, linéaire ou ramifié, ayant un nombre de carbone allant de 23 à 50, ou un mélange d'alcools primaires saturés de masse moléculaire moyenne en poids $\overline{Mw}$ allant de 340 à 718, et une polydispersité $\overline{Mw}/\overline{Mn}$ voisine de 1,
- soit un époxyalcane $C_nH_{2n}O$ dans lequel n va de 23 à 50 ou un mélange d'époxyalcanes de masse moléculaire moyenne en poids $\overline{Mw}$ allant de 338 à 716 ;

$R^1$ représente un radical hydrocarboné aliphatique, linéaire ou ramifié, ayant un nombre d'atomes de carbone allant de 1 à 12, et de préférence égal à 1,4 ou 8,

x est un nombre entier égal à 1 ou 2.

2. Composition selon la revendication 1, **caractérisée en ce que** la réaction a lieu en présence d'un solvant.

3. Composition selon la revendication 2, **caractérisée en ce qu'**elle est préparée selon les étapes suivantes qui consistent à fondre en milieu solvant et en chauffant un composant RA, puis à ajouter au milieu ainsi obtenu, porté à une température au moins égale à 80°C et, de préférence, comprise entre 90°C et 100°C, l'anhydride maléique en une durée allant de 15 minutes à 1 heure, puis ensuite à ajouter un oxyde de dialkylétain $(R^1)_2$ Sn = O en une durée allant de 15 minutes à 90 minutes à chauffer le milieu réactionnel ainsi obtenu à une température allant de 80°C à 120°C et, de préférence, comprise entre 90°C et 100°C pendant une durée allant de 15 minutes à 90 minutes et à éliminer l'eau formée ainsi que le solvant sous presssion réduite à une température allant de 80°C à 120°C et, de préférence, comprise entre 90°C et 100°C.

4. Composition selon l'une des revendications 1 à 3, **caractérisée en ce que** le rapport molaire anhydride maléique/ RA est au moins égal à 2 et, de préférence compris entre 2,5 et 4.

5. Composition selon l'une des revendications 1 à 3, **caractérisée en ce que** l'on utilise pour 1 mole d'anhydride maléique, au moins 0,50 mole d'oxyde de dialkylétain $(R^1)_2$Sn = O et, de préférence, une quantité allant de 0,75 mole à 0,85 mole.

6. Composition selon l'une des revendications 1 à 4, **caractérisée en ce que** le composé RA est un mélange d'alcools primaires saturés ayant une masse moléculaires moyenne en poids $\overline{Mw}$ allant de 375 à 700 et une polydispersité $\overline{Mw}/\overline{Mn}$ voisine de 1 et, de préférence un mélange d'alcools ayant une masse $\overline{Mw}$ égale à 460 ou 550.

7. Composition selon l'une des revendications 1 à 4, **caractérisée en ce que** le composé RA est un mélange d'époxyalcanes $C_nH_{2n}O$ ayant une masse moléculaire moyenne en poids $\overline{Mw}$ allant de 338 à 632 et, de préférence un mélange d'époxyalcanes ayant une masse $\overline{Mw}$ égale à 632.

8. Composition selon l'une des revendications 1 à 3 et 6, **caractérisée en ce que** l'oxyde de dialkylétain est l'oxyde de dibutylétain.

9. Composition selon la revendication 1, **caractérisée en ce que** l'on utilise un mélange de trichlorure de monoalkylétain $R^1SnCl_3$ et de dichlorure de dialkylétain $(R^1)_2SnCl_2$.

10. Composition selon l'une des revendications 1 à 9, **caractérisée en ce que** la réaction a lieu en présence d'eau.

11. Composition selon l'une des revendications 1 à 10, **caractérisée en ce que** l'on utilise, en outre, un costabilisant.

12. Composition selon la revendication 11 **caractérisée en ce que** le costabilisant est une zéolithe.

13. Procédé pour obtenir une composition de maléates d'organoétain de poids moléculaire élevé, **caractérisé en ce que** l'on fait réagir un composant RA avec l'anhydride maléique, puis que l'on met en contact le milieu réactionnel ainsi obtenu avec au moins un oxyde de dialkyl étain $(R^1)_2$ Sn = O ou avec au moins un chlorure d'alkylétain $(R^1)_x SnCl_{4-x}$, étant donné que :

RA représente

- soit un alcool ROH dans lequel R représente un radical hydrocarboné aliphatique, linéaire ou ramifié, ayant un nombre de carbone allant de 23 à 50 ou un mélange d'alcools primaires saturés de masse moléculaire moyenne en poids $\overline{Mw}$ allant de 340 à 718, et une polydispersité $\overline{Mw}/\overline{Mn}$ voisine de 1,
- soit un époxyalcane $C_nH_{2n}O$ dans lequel n va de 23 à 50 ou un mélange d'époxyalcanes de masse moléculaire moyenne en poids $\overline{Mw}$ allant de 338 à 716 ;

$R^1$ représente un radical hydrocarboné aliphatique, linéaire ou ramifié, ayant un nombre d'atomes de carbone allant de 1 à 12, et de préférence égal à 1,4 ou 8,
x est un nombre entier égal à 1 ou 2.

**14.** Procédé selon la revendication 13, **caractérisé en ce que** l'on opère en milieu solvant.

**15.** Procédé selon la revendication 14, **caractérisé en ce que** l'on dissout en milieu solvant et en chauffant, un composant RA, puis que l'on ajoute au milieu ainsi obtenu, porté à une température au moins égale à 80°C et, de préférence, comprise entre 90°C et 100°C, l'anhydride maléique en une durée allant de 15 minutes à 1 heure, puis ensuite que l'on ajoute un oxyde de dialkylétain $(R^1)_2$ Sn = O en une durée allant de 15 minutes à 90 minutes ; que l'on chauffe le milieu réactionnel ainsi obtenu à une température allant de 80°C à 120°C et, de préférence, comprise entre 90°C et 100°C pendant une durée allant de 15 minutes à 90 minutes et que l'on élimine l'eau formée ainsi que le solvant sous presssion réduite à une température allant de 80°C à 120°C et, de préférence, comprise entre 90°C et 100°C.

**16.** Procédé selon l'une des revendications 13 à 15, **caractérisé en ce que** le rapport molaire anhydride maléique/ RA est au moins égal à 2 et, de préférence compris entre 2,5 et 4.

**17.** Procédé selon l'une des revendications 13 à 15, **caractérisé en ce que** l'on utilise pour 1 mole d'anhydride maléique, au moins 0,50 mole d'oxyde de dialkylétain $(R^1)_2$Sn = O et, de préférence, une quantité allant de 0,75 mole à 0,85 mole.

**18.** Procédé selon l'une des revendications 13 à 16, **caractérisé en ce que** le composé RA est un mélange d'alcools primaires saturés ayant une masse moléculaire moyenne en poids $\overline{Mw}$ allant de 375 à 700 et une polydispersité $\overline{Mw}/\overline{Mn}$ voisine de 1 et, de préférence un mélange d'alcools ayant une masse $\overline{Mw}$ égale à 460 ou 550.

**19.** Procédé selon l'une des revendications 13 à 16, **caractérisé en ce que** le composé RA est un mélange d'époxyalcanes $C_nH_{2n}O$ ayant une masse moléculaire moyenne en poids $\overline{Mw}$ allant de 338 à 632 et, de préférence un mélange d'époxyalcanes ayant une masse $\overline{Mw}$ égale à 632.

**20.** Procédé selon l'une des revendications 13 à 15 et 17, **caractérisé en ce que** l'oxyde de dialkylétain est l'oxyde de dibutylétain.

**21.** Procédé selon la revendication 13, **caractérisé en ce que** l'on opère avec un mélange de trichlorure de monoalkylétain $R^1SnCl_3$ et de dichlorure de dialkylétain $(R^1)_2SnCl_2$.

**22.** Procédé selon l'une des revendications 13 à 21, **caractérisé en ce que** l'on opère en présence d'eau.

**23.** Procédé selon l'une des revendications 13 à 22, **caractérisé en ce que** l'on opère en présence d'un costabilisant.

**24.** Procédé selon la revendication 23, **caractérisé en ce que** le costabilisant est une zéolithe.

**25.** Composition de polymère thermoplastique stabilisée et lubrifiée renfermant une composition de maléates d'organoétain de poids moléculaire élevé selon l'une des revendications 1 à 12.

**26.** Composition selon la revendication 25, **caractérisé en ce que** le polymère thermoplastique est un homo- ou copolymère du chlorure de vinyle.

**27.** Article rigide extrudé compact, **caractérisé en ce qu'**il est formé à partir d'une composition de polymère thermoplastique selon l'une des revendications 25 et 26.

**Patentansprüche**

**1.** Zusammensetzung von Organozinnmaleaten mit hohem Molekulargewicht, die durch Umsetzen eines Bestandteils RA mit Maleinsäureanhydrid und anschließendes Inkontaktbringen des so erhaltenen Reaktionsmediums mit mindestens einem Dialkylzinnoxid $(R^1)_2$Sn=O oder mit mindestens einem Alkylzinnchlorid $(R^1)_xSnCl_{4-x}$ hergestellt wird, wobei bedeuten:

- RA

  - einen Alkohol ROH, worin R einen geradkettigen oder verzweigten aliphatischen Kohlenwasserstoffrest bedeutet, der 23 bis 50 Kohlenstoffatome enthält, oder ein Gemisch gesättigter primärer Alkohole mit einem Gewichtsmittel des Molekulargewichts $\overline{Mw}$ im Bereich von 340 bis 718 und einem Polymolekularitätsindex $\overline{Mw}$ / $\overline{Mn}$ von etwa 1, oder
  - ein Epoxyalkan $C_nH_{2n}O$, worin n im Bereich von 23 bis 50 liegt, oder ein Gemisch von Epoxyalkanen mit einem Gewichtsmittel des Molekulargewichts $\overline{Mw}$ im Bereich von 338 bis 716,

- $R^1$ einen geradkettigen oder verzweigten aliphatischen Kohlenwasserstoffrest, der 1 bis 12 Kohlenstoffatome und vorzugsweise 1, 4 oder 8 Kohlenstoffatome enthält,
- x die ganze Zahl 1 oder 2.

**2.** Zusammensetzung nach Anspruch 1, **dadurch gekennzeichnet, daß** die Umsetzung in Gegenwart eines Lösemittels stattfindet.

**3.** Zusammensetzung nach Anspruch 2, **dadurch gekennzeichnet, daß** sie nach den folgenden Verfahrensschritten hergestellt wird, die darin bestehen, in einem Lösemittelmedium und unter Erhitzen einen Bestandteil RA zu schmelzen, anschließend zu dem so erhaltenen Medium, das bei einer Temperatur von mindestens 80 °C und vorzugsweise im Bereich von 90 bis 100 °C gehalten wird, Maleinsäureanhydrid während eines Zeitraums von 15 min bis 1 h zuzugeben, dann ein Dialkylzinnoxid $(R^1)_2Sn{=}O$ während eines Zeitraums von 15 bis 90 min zuzugeben, das so erhaltene Reaktionsmedium während eines Zeitraums von 15 bis 90 min auf eine Temperatur von 80 bis 120 °C und vorzugsweise im Bereich von 90 bis 100 °C zu erwärmen und das dabei gebildete Wasser sowie das Lösemittel unter vermindertem Druck bei einer Temperatur von 80 bis 120 °C und vorzugsweise im Bereich von 90 bis 100 °C zu entrernen.

**4.** Zusammensetzung nach einem der Ansprüche 1 bis 3, **dadurch gekennzeichnet, daß** das Molverhältnis Maleinsäureanhydrid/RA mindestens 2 beträgt und vorzugsweise im Bereich von 2,5 bis 4 liegt.

**5.** Zusammensetzung nach einem der Ansprüche 1 bis 3, **dadurch gekennzeichnet, daß** pro 1 Mol Maleinsäureanhydrid mindestens 0,50 mol und vorzugsweise 0,75 bis 0,85 mol Dialkylzinnoxid $(R^1)_2Sn{=}O$ verwendet werden.

**6.** Zusammensetzung nach einem der Ansprüche 1 bis 4, **dadurch gekennzeichnet, daß** es sich bei der Verbindung RA um ein Gemisch gesättigter primärer Alkohole mit einem Gewichtsmittel des Molekulargewichts $\overline{Mw}$ im Bereich von 375 bis 700 und einem Polymolekularitätsindex $\overline{Mw}$ / $\overline{Mn}$ von etwa 1 und vorzugsweise ein Gemisch von Alkoholen mit einem Gewichtsmittel $\overline{Mw}$ von 460 oder 550 handelt.

**7.** Zusammensetzung nach einem der Ansprüche 1 bis 4, **dadurch gekennzeichnet, daß** es sich bei der Verbindung RA um ein Gemisch von Epoxyalkanen $C_nH_{2n}O$ mit einem Gewichtsmittel des Molekulargewichts $\overline{Mw}$ im Bereich von 338 bis 632 und vorzugsweise ein Gemisch von Epoxyalkanen mit einem Gewichtsmittel des Molekulargewichts $\overline{Mw}$ von 632 handelt.

**8.** Zusammensetzung nach einem der Ansprüche 1 bis 3 und 6, **dadurch gekennzeichnet, daß** es sich bei dem Dialkylzinnoxid um Dibutylzinnoxid handelt.

**9.** Zusammensetzung nach Anspruch 1, **dadurch gekennzeichnet, daß** ein Gemisch aus Monoalkylzinntrichlorid $R^1SnCl_3$ und Dialkylzinndichlorid $(R^1)_2SnCl_2$ verwendet wird.

**10.** Zusammensetzung nach einem der Ansprüche 1 bis 9, **dadurch gekennzeichnet, daß** die Umsetzung in Gegenwart von Wasser durchgeführt wird.

**11.** Zusammensetzung nach einem der Ansprüche 1 bis 10, **dadurch gekennzeichnet, daß** außerdem ein Costabilisierungsmittel verwendet wird.

**12.** Zusammensetzung nach Anspruch 11, **dadurch gekennzeichnet, daß** es sich bei dem Costabilisierungsmittel um einen Zeolith handelt.

**13.** Verfahren zur Herstellung einer Zusammensetzung von Organozinnmaleaten mit hohem Molekulargewicht, **da-**

**durch gekennzeichnet, daß** ein Bestandteil RA mit Maleinsäureanhydrid umgesetzt wird und daß das so erhaltene Reaktionsmedium mit mindestens einem Dialkylzinnoxid $(R^1)_2Sn=O$ oder mit mindestens einem Alkylzinnchlorid $(R^1)_xSnCl_{4-x}$ in Kontakt gebracht wird, wobei bedeuten:

- RA

  - einen Alkohol ROH, worin R einen geradkettigen oder verzweigten aliphatischen Kohlenwasserstoffrest bedeutet, der 23 bis 50 Kohlenstoffatome enthält, oder ein Gemisch gesättigter primärer Alkohole mit einem Gewichtsmittel des Molekulargewichts $\overline{Mw}$ im Bereich von 340 bis 718 und einem Polymolekularitätsindex $\overline{Mw}/\overline{Mn}$ von etwa 1, oder
  - ein Epoxyalkan $C_nH_{2n}O$, worin n im Bereich von 23 bis 50 liegt, oder ein Gemisch von Epoxyalkanen mit einem Gewichtsmittel des Molekulargewichts $\overline{Mw}$ im Bereich von 338 bis 716,

- $R^1$ einen geradkettigen oder verzweigten aliphatischen Kohlenwasserstoffrest, der 1 bis 12 Kohlenstoffatome und vorzugsweise 1, 4 oder 8 Kohlenstoffatome enthält,
- x die ganze Zahl 1 oder 2.

14. Verfahren nach Anspruch 13, **dadurch gekennzeichnet, daß** in einem Lösemittelmedium gearbeitet wird.

15. Verfahren nach Anspruch 14, **dadurch gekennzeichnet, daß** in einem Lösemittelmedium und unter Erhitzen ein Bestandteil RA geschmolzen wird, anschließend zu dem so erhaltenen Medium, das bei einer Temperatur von mindestens 80 °C und vorzugsweise im Bereich von 90 bis 100 °C gehalten wird, Maleinsäureanhydrid während eines Zeitraums von 15 min bis 1 h zugegeben wird, dann ein Dialkylzinnoxid $(R^1)_2Sn=O$ während eines Zeitraums von 15 bis 90 min zugegeben wird, das so erhaltene Reaktionsmedium während eines Zeitraums von 15 bis 90 min auf eine Temperatur von 80 bis 120 °C und vorzugsweise im Bereich von 90 bis 100 °C erwärmt wird und das dabei gebildete Wasser sowie das Lösemittel unter vermindertem Druck bei einer Temperatur von 80 bis 120 °C und vorzugsweise im Bereich von 90 bis 100 °C entfernt werden.

16. Verfahren nach einem der Ansprüche 13 bis 15, **dadurch gekennzeichnet, daß** das Molverhältnis Maleinsäureanhydrid/RA mindestens 2 beträgt und vorzugsweise im Bereich von 2,5 bis 4 liegt.

17. Verfahren nach einem der Ansprüche 13 bis 15, **dadurch gekennzeichnet, daß** pro 1 Mol Maleinsäureanhydrid mindestens 0,50 mol und vorzugsweise 0,75 bis 0,85 mol Dialkylzinnoxid $(R^1)_2Sn=O$ verwendet werden.

18. Verfahren nach einem der Ansprüche 13 bis 16, **dadurch gekennzeichnet**, es sich bei der Verbindung RA um ein Gemisch gesättigter primärer Alkohole mit einem Gewichtsmittel des Molekulargewichts $\overline{Mw}$ im Bereich von 375 bis 700 und einem Polymolekularitätsindex $\overline{Mw}/\overline{Mn}$ von etwa 1 und vorzugsweise ein Gemisch von Alkoholen mit einem Gewichtsmittel $\overline{Mw}$ von 460 oder 550 handelt.

19. Verfahren nach einem der Ansprüche 13 bis 16, **dadurch gekennzeichnet, daß** es sich bei der Verbindung RA um ein Gemisch von Epoxyalkanen $C_nH_{2n}O$ mit einem Gewichtsmittel des Molekulargewichts $\overline{Mw}$ im Bereich von 338 bis 632 und vorzugsweise ein Gemisch von Epoxyalkanen mit einem Gewichtsmittel des Molekulargewichts $\overline{Mw}$ von 632 handelt.

20. Verfahren nach einem der Ansprüche 13 bis 15 und 17, **dadurch gekennzeichnet, daß** es sich bei dem Dialkylzinnoxid um Dibutylzinnoxid handelt.

21. Verfahren nach Anspruch 13, **dadurch gekennzeichnet, daß** ein Gemisch aus Monoalkylzinntrichlorid $R^1SnCl_3$ und Dialkylzinndichlorid $(R^1)_2SnCl_2$ verwendet wird.

22. Verfahren nach einem der Ansprüche 13 bis 21, **dadurch gekennzeichnet, daß** in Gegenwart von Wasser gearbeitet wird.

23. Verfahren nach einem der Ansprüche 13 bis 22, **dadurch gekennzeichnet, daß** in Gegenwart eines Costabilisierungsmittels gearbeitet wird.

24. Verfahren nach Anspruch 23, **dadurch gekennzeichnet, daß** es sich bei dem Costabilisierungsmittel um einen Zeolith handelt.

25. Gleitmittel enthaltende, stabilisierte thermoplastische Polymerzusammensetzung, die eine Zusammensetzung von Organozinnmaleaten mit hohem Molekulargewicht nach einem der Ansprüche 1 bis 12 enthält.

26. Zusammensetzung nach Anspruch 25, **dadurch gekennzeichnet, daß** es sich bei dem thermoplastischen Polymer um ein Homo- oder Copolymer von Vinylchlorid handelt.

27. Dichter, extrudierter, steifer Gegenstand, **dadurch gekennzeichnet, daß** er aus einer thermoplastischen Polymerzusammensetzung nach einem der Ansprüche 25 und 26 hergestellt ist.

**Claims**

1. Composition containing high molecular weight organotin maleates as obtained by the reaction of a component RA with maleic anhydride and then bringing the reaction mixture thus obtained into contact with at least one dialkyltin oxide $(R^1)_2Sn=O$ or with at least one alkyltin chloride $(R^1)_xSnCl_{4-x}$, given that:

   RA represents

   - either an alcohol ROH in which R represents a linear or branched aliphatic hydrocarbon radical having a carbon number ranging from 23 to 50 or a mixture of saturated primary alcohols with a weight-average molecular mass $\overline{Mw}$ ranging from 340 to 718 and a polydispersity $\overline{Mw}/\overline{Mn}$ in the region of 1,
   - or an epoxyalkane $C_nH_{2n}O$ in which n ranges from 23 to 50 or a mixture of epoxyalkanes with a weight-average molecular mass $\overline{Mw}$ ranging from 338 to 716;

   $R^1$ represents a linear or branched aliphatic hydrocarbon radical having a number of carbon atoms ranging from 1 to 12 and preferably equal to 1, 4 or 8,
   x is an integer equal to 1 or 2.

2. Composition according to Claim 1, **characterized in that** the reaction takes place in the presence of a solvent.

3. Composition according to Claim 2, **characterized in that** it is prepared according to the following stages which consist in dissolving a component RA in solvent medium while heating, in then adding to the mixture thus obtained, brought to a temperature of at least 80°C and preferably of between 90°C and 100°C, maleic anhydride over a period of time ranging from 15 minutes to 1 hour and in then subsequently adding a dialkyltin oxide $(R^1)_2Sn=O$ over a period of time ranging from 15 minutes to 90 minutes; in heating the reaction mixture thus obtained at a temperature ranging from 80°C to 120°C and preferably of between 90°C and 100°C for a period of time ranging from 15 minutes to 90 minutes and in removing the water formed and the solvent under reduced pressure at a temperature ranging from 80°C to 120°C and preferably of between 90°C and 100°C.

4. Composition according to one of Claims 1 to 3, **characterized in that** the maleic anhydride/RA molar ratio is at least 2 and preferably between 2.5 and 4.

5. Composition according to one of Claims 1 to 3, **characterized in that** at least 0.50 mol of dialkyltin oxide $(R^1)_2Sn=0$ and preferably an amount ringing from 0.75 mol to 0.85 mol is used per 1 mol of maleic anhydride.

6. Composition according to one of Claims 1 to 4, **characterized in that** the compound RA is a mixture of saturated primary alcohols having a weight-average molecular mass $\overline{Mw}$ ranging from 375 to 700 and a polydispersity $\overline{Mw}/\overline{Mn}$ in the region of 1 and preferably a mixture of alcohols having a mass $\overline{Mw}$ of 460 or 550.

7. Composition according to one of Claims 1 to 4, **characterized in that** the compound RA is a mixture of epoxyalkanes $C_nH_{2n}O$ having a weight-average molecular mass $\overline{Mw}$ ranging from 338 to 632 and preferably a mixture of epoxyalkanes having amass $\overline{Mw}$ of 632.

8. Composition according to one of Claims according to 1 to 3 and 6, **characterized in that** the dialkyltin oxide is dibutyltin oxide.

9. Composition according to Claim 1, **characterized in that** a mixture of monoalkyltin trichloride $R^1SnCl_3$ and of dialkyltin dichloride $(R^1)_2SnCl_2$ is used.

10. Composition according to one of Claims 1 to 9, **characterized in that** the reaction takes place in the presence of water.

11. Composition according to one of Claims 1 to 10, **characterized in that** a costabilizer is additionally used.

12. Composition according to Claim 11, **characterized in that** the costabilizer is a zeolite.

13. Process for producing a composition containing high molecular weight organotin maleates, **characterized in that** a component RA is reacted with maleic anhydride and that the reaction mixture thus obtained is then brought into contact with at least one dialkyltin oxide $(R^1)_2Sn=O$ or with at least one alkyltin chloride $(R^1)_xSnCl_{4-x}$, given that:

   RA represents

   - either an alcohol ROH in which R represents a linear or branched aliphatic hydrocarbon radical having a carbon number ranging from 23 to 50 or a mixture of saturated primary alcohols with a weight-average molecular mass $\overline{Mw}$ ranging from 340 to 718 and a polydispersity $\overline{Mw}/\overline{Mn}$ in the region of 1,
   - or an epoxyalkane $C_nH_{2n}O$ in which n ranges from 23 to 50 or a mixture of epoxyalkanes with a weight-average molecular mass $\overline{Mw}$ ranging from 338 to 716;

   $R^1$ represents a linear or branched aliphatic hydrocarbon radical having a number of carbon atoms ranging from 1 to 12 and preferably equal to 1, 4 or 8,
   x is an integer equal to 1 or 2.

14. Process according to Claim 13, **characterized in that** the operation is carried out in solvent medium.

15. Process according to Claim 14, **characterized in that** a component RA is dissolved in solvent medium while heating, that maleic anhydride is then added over a period of time ranging from 15 minutes to 1 hour to the mixture thus obtained, which has been brought to a temperature of at least 80°C and preferably of between 90°C and 100°C, and that a dialkyltin oxide $(R^1)_2Sn=O$ is then subsequently added over a period of time ranging from 15 minutes to 90 minutes; that the reaction mixture thus obtained is heated at a temperature ranging from 80°C to 120°C and preferably of between 90°C and 100°C for a period of time ranging from 15 minutes to 90 minutes and that the water formed and the solvent are removed under reduced pressure at .a temperature ranging from 80°C to 120°C and preferably of between 90°C and 100°C.

16. Process according to one of Claims 13 to 15, **characterized in that** the maleic anhydride/RA molar ratio is at least 2 and preferably between 2.5 and 4.

17. Process according to one of Claims 13 to 15, **characterized in that** at least 0.50 mol of dialkyltin oxide $(R^1)_2Sn=0$ and preferably an amount ranging from 0.75 mol to 0.85 mol is used per 1 mol of maleic anhydride.

18. Process according to one of Claims 13 to 16, **characterized in that** the compound RA is a mixture of saturated primary alcohols having a weight-average molecular mass $\overline{Mw}$ ranging from 375 to 700 and a polydispersity $\overline{Mw}/\overline{Mn}$ in the region of 1 and preferably a mixture alcohols having a mass $\overline{Mw}$ of 460 or 550.

19. Process according to one of Claims 13 to 16, **characterized in that** the compound RA is a mixture of epoxyalkanes $C_nH_{2n}O$ having a weight-average molecular mass $\overline{Mw}$ ranging from 338 to 632 and preferably a mixture of epoxyalkanes having a mass $\overline{Mw}$ of 632.

20. Process according to one of Claims 13 to 15 and 17, **characterized in that** the dialkyltin oxide is dibutyltin oxide.

21. Process according to Claim 13, **characterized in that** the operation is carried out with a mixture of monoalkyltin trichloride $R^1SnCl_3$ and of dialkyltin dichloride $(R^1)_2SnCl_2$.

22. Process according to one of Claims 13 to 21, **characterized in that** the operation is carried out in the presence of water.

23. Process according to one of Claims 13 to 22, **characterized in that** the operation is carried out in the presence of a costabilizer.

24. Process according to Claim 23, **characterized in that** the costabilizer is a zeolite.

25. Stabilized and lubricated thermoplastic polymer composition including a composition containing high molecular weight organotin maleates according to one of Claims 1 to 12.

26. Composition according to Claim 25, **characterized in that** the thermoplastic polymer is a homo- or copolymer of vinyl chloride.

27. Compact extruded rigid article, **characterized in that** it is formed from a thermoplastic polymer composition according to either of Claims 25 and 26.

FIGURE 1